# EUROPEAN PATENT SPECIFICATION

(11) **EP 1 321 139 B1**
(45) Date of publication and mention of the grant of the patent: **23.08.2006**
(21) Application number: 01130638.8
(22) Date of filing: 21.12.2001
(51) Int. Cl.: A61K 31/165, A61P 25/04, A61P 25/02

(54) **2-Indanylamino deratives for the therapy of chronic, acute or inflammatory pain**
2-Indanylaminoderivate zur Therapie von chronischen, akuten oder entzündlichen Schmerzen
Dérivés du 2-indanylamine pour la thérapie de douleurs chroniques, aigues ou inflammatoires

(43) Date of publication of application: 25.06.2003
(73) Proprietor: CHIESI FARMACEUTICI S.p.A., I-43100 Parma (IT)
(72) Inventor: Pietra, Claudio, 43100 Parma (IT); Villetti, Gino, 43100 Parma (IT)
(74) Representative: Minoja, Fabrizio

(56) References cited:
- EP-A- 0 615 749
- WO-A-00/76510
- WO-A-98/03472
- WO-A1-98/50044
- VILLETTI G ET AL: "Preclinical evaluation of CHF3381 as a novel antiepileptic agent." NEUROPHARMACOLOGY. ENGLAND JUN 2001, vol. 40, no. 7, June 2001 (2001-06), pages 866-878, XP001076807 ISSN: 0028-3908
- GANDOLFI O ET AL: "Anticonvulsant preclinical profile of CHF 3381: dopaminergic and glutamatergic mechanisms." PHARMACOLOGY, BIOCHEMISTRY, AND BEHAVIOR. UNITED STATES SEP 2001, vol. 70, no. 1, September 2001 (2001-09), pages 157-166, XP001076806 ISSN: 0091-3057
- PARSONS CHRIS G: "NMDA receptors as targets for drug action in neuropathic pain." EUROPEAN JOURNAL OF PHARMACOLOGY, vol. 429, no. 1-3, 2001, pages 71-78, XP010076802 ISSN: 0014-2999
- VILLETTI G. ET AL: 'Antinociceptive activity of the N-methyl-D-aspartate receptor antagonist N-(2-indanyl)-glycinamide hydrochloride (CHF3381) in experimental models of inflammatory and neuropathic pain' J. PHARM. EXP. THER. vol. 306, no. 2, 2003, pages 804 - 814
- CHAPLAN S.R. ET AL: 'Efficacy of spinal NMDA receptor antagonism in formalin hyperalgesia and nerve injury evoked allodynia in the rat' J. PHARM. EXP. THER. vol. 280, no. 2, 1997, pages 829 - 238
- NÄSSTRÖM J. ET AL: 'Antinociceptive actions of different classes of excitatory amino acid receptor antagonists in mice' EUR. J. PHARM. vol. 212, 1992, pages 21 - 29
- 'Rethinking opioid equivalence' PAIN CLINICAL UPDATES no. 4, September 2002,

## Description

The present invention relates to the use of compounds represented by the general formula I: 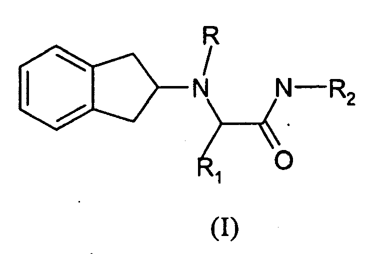 wherein:
- R: is hydrogen or C₁-C₄ alkyl groups;
- R₁: is hydrogen, alkyl or optionally acylated C₁-C₄ hydroxyalkyl;
- R₂: is hydrogen; alkyl; phenyl; phenylalkyl
and salts thereof for the manufacture of a medicament for the treatment of chronic pain.

Preferred compounds are those wherein:
- R: is H
- R₁: is H or alkyl
- R₂: is H or alkyl

In a more preferred embodiment, the invention relates to the use of 2-(2-indanylamino)-acetamide or [N-(2-indanyl)-glycinamide] for the treatment of chronic pain, in particular the pain associated with postherpetic neuralgia, trigeminal neuralgia, diabetic neuropathy and nerve destruction by the human immunodeficiency virus (HIV).

### Prior art

Chronic pain is a broad term generally defined as pain that persists beyond the usual course of an acute disease or beyond a reasonable time for an injury to heal or that recurs at intervals for months or years. Although it may present in many different forms and can vary significantly in etiology, clinical course and response, all type of chronic pain involve basic aberrations in somatosensory processing in the central and/or peripheral nervous system. Researchers generally identify at least three distinct categories of pain:
- *Nociceptive pain,* or somatic pain, which is the normal physiological response to pain. This form of pain is relayed to the central nervous system (CNS) via nociceptors, which are primary afferent nerve fibers located in peripheral tissues and organs. Examples include pain caused by acute trauma (before inflammation is established) and pain caused by a cancerous tumor that invades and stretches an organ.
- *Inflammatory pain* which is triggered by nociceptive afferents that become irritated when surrounded by inflamed tissue. Inflammatory pain is commonly observed among patients with arthritis, patients experiencing inflammation following back injuries and cancer patients who present an inflammation surrounding an obstructive tumor.
- *Neuropathic pain* which occurs specifically from nerve injury and may persist even after the injured nerve is healed. It is considered particularly insidious because most afflicted patients are refractory to standard analgesic drugs. Neuropathic pain may be present in a significant proportion of patients with chronic low-back pain or cancer pain. It is also the etiology of pain associated with postherpetic neuralgia, trigeminal neuralgia, diabetic neuropathy and nerve destruction by the human immunodeficiency virus (HIV).

There are several factors that can cause, perpetuate or exacerbate chronic pain. First, of course, the patient may simply have a disease such as arthritis, cancer, migraine headaches, fibromyalgia and diabetic neuropathy, that is characteristically painful and for which there is presently no cure. Second, there may be secondary perpetuating factors that are initiated by a bodily disease and persist after that disease has resolved. Examples include damaged sensory nerves, sympathetic efferent activity and painful reflex muscle contraction. Finally, a variety of physiological conditions can exacerbate or even cause pain.

The current pharmacological treatment, based on analgesic, anticonvulsant and antidepressant drugs, do not offer complete efficacy and many have troublesome side effects.

Therefore, more effective and tolerable analgesic therapies are needed, a void that experts believe can be filled only by agents that feature novel and more-specific mechanisms of action.

A corollary to the unmet need for more-specific drugs is the need for effective and safe agents that have been developed precisely for the treatment of chronic pain associated with symptoms of neuropathy (neuropathic pain) such as diabetic neuropathy or postherpetic neuralgia.

The size of the afflicted neuropathic pain population is significant, albeit unknown, especially in chronic cancer and low-back pain.

Therefore, it would be particular advantageous to provide agents that, due to the greater selectivity for highly specific targets, will effectively eliminate said pain symptoms without affecting the body's normal physiology.

Compounds of formula (I) have been described for the first time in WO 98/03472, in the name of the applicant, among a number of α-amino-acid amide derivatives investigated as potential therapeutic agents for the treatment of chronic neurodegenerative diseases, such as Alzheimer' disease, various forms of dementia, Parkinson's disease, Huntington's disease or acute neurodegenerative impairments such as stroke and head injuries and for the treatment of epilepsy and depression.

Said compounds turned out to be provided of anti-convulsivant activity in the rat MES model and in the chemical model of tonic convulsions induced by bicuculline.

Villetti et al (Neuropharmacology 2001, 40, 866) in a study aimed at closely investigating its antiepileptic properties, have reported in particular that N-2(indanyl)-glycinamide hydrochloride (indicated hereinafter with the experimental code CHF 3381) is very effective in seizure models against maximal electroshock seizures, picrotoxin- and N-methyl-D-aspartate (NMDA)-induced hind limb tonic extension but is a weaker antagonist of 4-aminopyridine- and bicuculline-induced tonic seizures and is ineffective against pentylentetrazole- and picrotoxin-induced clonic seizures. Moreover, CHF 3381 was reported to antagonize the behavioral effects and the lethality of systematically administered NMDA, indicating that the compound may act as a functional NMDA antagonist. In keeping with this idea, CHF 3381 weakly displaced [³H]-TPC from binding to NMDA receptors channels (*Ki =* 8.8 µM).

EP-A-0 615 749 discloses the use of nontoxic antagonists for the NMBA receptor for the treatment of chronic pain, such as neuropathic pain, and acute persistent pain that is related to inflammation of injured body tissues.

### Disclosure of the invention

Now it has been found that CHF 3381 exhibits a unique dual inhibiting activity towards MAO (mono amino oxidase) and ion channel associated to NMDA receptors and, by virtue of such dual action, it possess an analgesic activity in animal models of acute and chronic pain.

Indeed, CHF 3381 turned out to be effective in some pharmacological models of the pain-state. These models inquire three categories of pain, i.e. chronic, inflammatory and acute pain and they are widely used to asses the efficacy of analgesic agents.

In the formalin model of inflammatory pain, CHF 3381 clearly decreased the number of flinches during the early and late nociceptive phases both in mice and rats. In rats, at 100 mg/kg p.o., the highest dose tested, these effects were similar to those observed with morphine at 64 mg/kg p.o.. In mice, CHF3381 almost completely blocked both acute and tonic formalin-induced flinch responses at 100 mg/kg p.o. and 60 mg/kg i.p.. In another model of inflammatory pain, the carrageenan model, CHF 3381 provided a nearly complete reversal of thermal hyperalgesia induced by carrageenan at 100 mg/kg p.o. and 60 mg/kg i.p.. In a rat model of chronic pain (ligature of the sciatic nerve) CHF3381 at 10-60 mg/kg i.p. reversed the thermal hyperalgesia without effects on motor reflexes.

It has also been demonstrated that CHF 3381 induces sedation and ataxia at doses substantially higher than those endowed with an antihyperalgesic effect indicating that its analgesic activity is not compromised by serious side-effects.

In view of these findings, compounds of formula (I) can be advantageously used for the preparation of pharmaceutical compositions for the management of any form of chronic pain, in particular for the treatment of neuropathic pain, i.e the pain associated with postherpetic neuralgia, trigeminal neuralgia, diabetic neuropathy and nerve destruction by the human immunodeficiency virus (HIV).

### Detailed description of the invention

The present invention relates to the use of compounds represented by the general formula I: 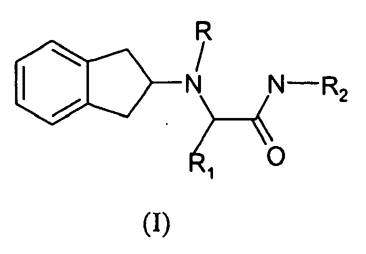 wherein:
- R: is hydrogen or C₁-C₄ alkyl groups;
- R₁: is hydrogen, alkyl or optionally acylated C₁-C₄ hydroxyalkyl;
- R₂: is hydrogen; alkyl; phenyl; phenylalkyl
and salts thereof for the preparation of medicaments for the treatment of any form of chronic pain, in particular for the treatment of neuropathic pan, i.e. the pain associated with postherpetic neuralgia, trigeminal neuralgia, diabetic neuropathy and nerve destruction by the human immunodeficiency virus (HIV).

Preferred compounds are those wherein:
- R: is H
- R₁: is H or alkyl
- R₂: is H or alkyl

An alkyl group if not otherwise specified is a C₁-C₁₀ alkyl group such as methyl, ethyl, n-propyl, isopropyl, n-butyl, isobutyl, n-pentyl, n-hexyl, n-heptyl, n-octyl, n-nonyl, 2-ethylpentyl, 1-ethylheptyl, 1-methyloctyl.

An acylated C₁-C₄ hydroxyalkyl group is acetyloxyalkyl, propanoyloxyalkyl, 2-methylpropanoyloxyalkyl, benzoyloxyalkyl group.

The most preferred compound is that wherein R, R₁ and R₂ are hydrogen [2-(2-indanylamino)acetamide].

For the envisaged therapeutic uses, compounds represented by formula I will be formulated in suitable pharmaceutical compositions.

Said compositions will typically contain 1 to 1000 mg of active ingredient, preferably 50 to 500 mg, more preferably 100 to 350 mg and will be administered one or more times a day depending on the disease and the conditions (weight, sex, age) of the patient.

The compositions will be prepared using conventional techniques and pharmaceutically acceptable excipients as described for example in Remington's Pharmaceutical Sciences Handbook, Mack. Pub., N.Y., USA, and will be administered by the oral, parenteral or rectal route. Examples of formulations comprise tablets, capsules, syrups, granulates, sterile injectable solutions or suspensions, suppositories and the like.

The following examples further illustrate the invention.

### Example 1 - Analgesic activity in the Chronic Constriction Injury model

The potential analgesic activity of CHF 3381 was evaluated the Chronic Constriction Injury (CCI) model described by Bennett et al (*Pain* 1988, 33: 87-107). Briefly, the rat left common sciatic nerve was exposed, and proximal to the sciatic trifurcation about 10 mm of nerve was freed of adhering tissue and four ligatures (4.0 silk ) were loosely tied around it with about 1 mm of spacing.

Two tests of hind limb withdrawal to thermal and cold stimuli were employed in this study. Each test was repeated on both the operated hind paw and the controlateral hind paw.

Rats were tested for thermal hyperalgesia using a commercial available analgesimeter (Plantar test, Ugo Basile, Comerio Italy) by applying heat stimulus (50W, 8V) directed onto the plantar surface of each hind paw, and the paw withdrawal latency (s) was determined. Four latency measurements were taken for each hind paw and averaged. The apparatus was calibrated to give a paw withdrawal latency of approximately 10 sec. The results were expressed as the difference score (DS) by subtracting the latency of the control side from the latency of the ligated side; if this difference was less than 1.5 sec, the animal was not included in the experimental groups. CHF 3381 (10-30-60 mg/kg intraperitoneally -i.p.-) or vehicle were administered to animals 14-21 days after ligation and hyperalgesia tested 30 min, 1 and 2 hours after treatment. CHF3381 reversed the thermal hyperalgesia produced by CCI in a dose- and time-dependent manner with a maximum effect at 60 min after the administration. A significant effect was observed at the 30 and 60 mg/kg doses; a non-significant trend towards an effect was observed at 10 mg/kg (Table 1).

Cold allodynia was assessed in operated rats, confining them into a clear plastic cylinders placed upon a metal floor chilled by an underlying water bath. A thermistor placed on the floor indicated a surface temperature of about 5°C. In this experimental condition, after the ligation operated rats respond by lifting the affected hind paw elevated above the floor. Sham-operated animals does not withdraw the paw from the cold surface at any time. A maximum cut-off time of 20 sec was set to avoid any possible interference with the sensitivity of the animal to respond to subsequent exposure to the cold stimulus. Animals were pre-screened twice with 20 min interval between tests, in order to select for animals displaying clear signs of cold allodynia, i.e. animals with a paw withdrawal latency on the ligated side of < 13 s in both trials.

The animals were then assigned to groups consisting of at least 10 animals per group. CHF 3381 (10-30-60 mg/kg i.p.) or vehicle were administered to animals 7-14 days after ligation and cold allodynia tested 30 min, 1 and 2 hours after treatment. CHF 3381 also reversed cold allodynia produced by CCI. This effect was again observed to be both time- and dose-dependent with the results generally concurring with those from the thermal hyperalgesia studies. The effect was maximum at 60-120 min after the administration and significant at all the three tested doses (Table 1).

**Table 1 - Dose-response effect of CHF 3381 in the CCI model. The data are shown as means ± S.E.M.**

| | Hyperalgesia | Allodynia | |
|---|---|---|---|
| | | 60 min | 120 min |
| | Difference score (DS) | (sec) | (sec) |
| Control (vehicle) | 1.90±0.38 | 5.01±1.68 | 4.30±0.86 |
| CHF 3381 | | | |
| 10 mg/kg i.p. | 1.41±0.36 | 9.21±1.53* | 7.49±1.93 |
| 30 mg/kg i.p. | 0.83±0.35* | 10.04±1.99** | 8.79±1.95* |
| 60 mg/kg i.p. | 0.68±0.14** | 12.13±1.34** | 14.56±1.5** |

| | | | |
|---|---|---|---|
| ***P* <.01, **P*<.05 vs. vehicle-treated animals (n=10-15) | | | |

### Example 2 - Analgesic activity in the mice paw formalin model

The antihyperalgesic effect of CHF3381 was studied in the inflammatory pain model induced by formalin.

The mice paw formalin test was performed as described by Wheeler-Aceto et al. (*Psychopharmacology* 104:35-44, 1991). Briefly, the day before the formalin injection, mice were placed individually into clear plastic cylinders for 30 minutes of adaptation. The day of testing 20 µl of 1% formalin was injected into the plantar surface of the left hind paw and the animals were again placed into the plastic cylinder for the behavioural observation. The amount of time, in seconds, the animals spent licking and flinching (L/F) the injected paw for the first 5 min (early phase), and then from 10 to 40 min(late phase) after formalin injection, was used as measurement of intensity of pain. CHF 3381 10-100 mg/kg i.p. and 25-200 mg/kg p.o. or the corresponding vehicles, were administered 15 and 30 min before formalin injection, respectively.

In the vehicle-treated group, subcutaneous injection of formalin induced marked spontaneous nociceptive behavior. CHF 3381 induced a dose-related inhibition of the nociceptive responses in both phases either after oral and intraperitoneal treatment. After CHF 3381 intraperitoneal treatment, the antihyperalgesic effect was significant at 30, 60 and 100 mg/kg, and at 10, 30, 60 and 100 mg/kg in the early and late phases, respectively. After oral treatment with CHF 3381, the antihyperalgesic effect was significant at 50, 100 and 200mg/kg, and at 25, 50, 100 and 200 mg/kg in the early and late phases, respectively (Table 2).

Moreover, the formalin test was used to examine whether tolerance develops with respect to the antihyperalgesic effect of CHF 3381 after chronic treatment in comparison with the standard opioid morphine. Briefly, mice were divided randomly into five groups (12 mice per group) and administered once daily for 8 days as follows: three groups with saline i.p., one group with CHF 3381 60 mg/kg i.p. and one group with morphine 20 mg/kg i.p. On ninth day these groups were treated in following way: one saline pre-treated group was treated with saline i.p (gl); two saline pre-treated group were treated with CHF 3381 30 mg/kg i.p.(g2) and with morphine 6 mg/kg i.p.(g3), respectively; the group pre-treated with CHF 3381 60 mg/kg was treated with CHF 3381 30mg/kg i.p.(g4) and the group pre-treated with morphine 20 mg/kg was treated with morphine 6 mg/kg i.p.(g5), a dose that was previously shown to be active in the formalin test. CHF 3381 and morphine were administered 15 and 30 min before formalin injection, respectively.

Morphine (6 mg/kg i.p.) antagonised both the early and late phases of the formalin response in chronic saline-treated animals. However, the same dose of morphine failed to show such actions in animals subjected to chronic morphine treatment. In contrast, CHF3381 (30 mg/kg i.p.) still demonstrated a comparable antihyperalgesic activity in mice given chronic administration of either CHF 3381 (60 mg/kg i.p.) or vehicle, indicating a lack of development of tolerance (Table 3).

**Table 2 - Mouse Formalin Test acute treatment. The data are shown as means ± S.E.M.**

| | Early phase | Late phase | | Early phase | Late phase |
|---|---|---|---|---|---|
| | (sec) | (sec) | | (sec) | (sec) |
| Control (vehicle) | 95±6 | 241±31 | Control (vehicle) | 100±8 | 186±36 |
| CHF 3381.01 (i.p.) | | | CHF 3381.01 (p.o.) | | |
| 10 mg/kg | 95±8 | 165±26** | 25 mg/kg | 99±8 | 92±25** |
| 30 mg/kg | 59±9 ** | 58±1** | 50 mg/kg | 72±7 * | 30±9** |
| 60 mg/kg | 32±7** | 17±5** | 100 mg/kg | 46±7** | 23±16** |
| 100 mg/kg | 17±4 ** | 2±2 ** | 200 mg/kg | 47±4 ** | 12±10** |

| | | | | | |
|---|---|---|---|---|---|
| ***P* <.01, **P*<.05 vs. vehicle-treated animals (n=10-15) | | | | | |

**Table 3 - Mouse Formalin Test 9 day treatment. The data are shown as means ± S.E.M.**

| Group Treatment | Early phase (sec) | Late phase (sec) |
|---|---|---|
| G1 saline | 102 ± 7 | 216 ± 33 |
| G2 CHF3381(30 mg/kg) | 53 ± 8** | 91 ± 21** |
| G3 Morphine 6 mg/kg | 52 ± 15** | 91 ± 21** |
| G4 CHF 3381 30 mg/kg | 56 ± 6 ** | 74 ± 21** |
| G5 Morphine 6 mg/kg | 95 ± 9# | 175 ± 22# |

| | | |
|---|---|---|
| ***P* <.01, **P*<.05 versus g1; ^{#}*P*<.05 versus g3 (n=12) | | |

### Example 3 - Analgesic activity in the carrageenan-induced thermal hyperalgesia model

In the carrageenan-induced thermal hyperalgesia model, male rats were habituated to the rat plantar test apparatus and thermal hyperalgesia was then assessed as described in the paragraph concerning the CCI model. Briefly, after baseline paw withdrawal latencies were determined, animals received an intraplantar injection of carrageenan (100 µl of a 20 mg/ml solution) into the right hind paw. Paw withdrawal latencies (PWL) were reassessed following the same protocol as above 2.5 hours after carrageenan. (this time point represented the start of peak hyperalgesia) to ascertain that hyperalgesia had developed. CHF 3381 (3-10-30-60 mg/kg i.p. and 10-30-60-100 mg/kg p.o.) was then administered 3 hours post carrageenan and paw withdrawal latencies were taken again at 3.5, 4 and 5 hours post carrageenan. Carrageenan induced a significant reduction of paw withdrawal latency in all animals at 2.5 hours following injection. This hyperalgesia was maintained in vehicle-treated animals for at least five hours after carrageenan. The i.p. and p.o. administration of CHF 3381 at 3 hours after carrageenan, dose-dependently antagonised the maintenance of thermal hyperalgesia with respective minimum effective doses of 10 mg/kg i.p. and 30 mg/kg p.o. (Table 4).

**Table 4 - Carrageenan-induced thermal hyperalgesia. The data are shown as mean ± S.E.M. (n= 10-12)**

| | Time after carrageenan (h) | | | | |
|---|---|---|---|---|---|
| | 0 | 2.5 | 3.5 | 4.0 | 5.0 |
| | (sec) | (sec) | (sec) | (sec) | (sec) |
| Vehicle | 11.4±0.57 | 4.0±0.50 | 3.9±0.30 | 4.7±0.37 | 6.3±0.50 |
| CHF 3381 i.p. | | | | | |
| 3 mg/kg | 11.2±0.92 | 4.4±0.50 | 5.1±0.60 | 6.7±0.64 | 8.3±0.50 |
| 10 mg/kg | 11.8±0.49 | 5.1±0.50 | 7.4±0.50* | 7.8±0.66* | 8.4±0.80 |
| 30 mg/kg | 11.3±0.84 | 4.7±0.60 | 6.8±0.70** | 8.8±0.80** | 8.1±0.80 |
| 60 mg/kg | 11.4±0.85 | 4.1±0.50 | 8.0±1.20** | 10.4±1.39** | 10.7±0.90** |
| Vehicle | 11.8±0.75 | 4.98±0.77 | 5.75±0.78 | 5.06±0.52 | 6.36±0.53 |
| CHF 3381 p.o. | | | | | |
| 10 mg/kg | 12.96±1.01 | 4.79±0.71 | 6.65±1.24 | 6.90±0.61 | 7.29±0.83 |
| 30 mg/kg | 11.98±0.71 | 5.78±0.93 | 7.38±0.65 | 7.61±0.59* | 9.04±0.85 |
| 60 mg/kg | 11.98±0.67 | 5.22±0.58 | 8.68±1.16 | 8.20±0.85 ** | 8.34±0.81 |
| 100 mg/kg | 13.24±0.75 | 5.52±0.96 | 10.31±1.20* | 8.72±0.75** | 9.34±0.85* |

| | | | | | |
|---|---|---|---|---|---|
| ***P* <.01, **P*<.05 vs. vehicle-treated animals | | | | | |

### Example 4 - Analgesic activity on the hot-plate model

The effect of CHF 3381 was studied in acute pain with hot-plate test described by Eddy et al (1953). The test was performed on an electrically heated and thermostatically controlled copper surface, set to a temperature of 55 or 51 °C with mice and rats, respectively.

The animals were confined to the hot plate by a transparent observation chamber and the latency to the response consisting of licking of the hind paws, was measured. A cut-off period of 60 sec was used to avoid tissue damage.

The time of peak effect was determined before performing the dose-response curves and was shown to be 15 min after i.p. administration both in mice and rats. After i.p. administration, CHF 3381 (30-45-60 and 100 mg/kg in mouse; 30-37-45 and 60 mg/kg in rats), produced a significant and dose-dependent increase in the latency of the hindpaw licking response compared to vehicle-treated animals (Table 5).

**Table 5 - Hot-plate test.**

| The data are shown as means ± S.E.M. (n=20). | | | |
|---|---|---|---|
| | Mice (sec) | | Rats (sec) |
| Control (vehicle) | 17.3±1.28 | Control (vehicle) | 20.4±1.13 |
| CHF 3381.01 (i.p.) | | CHF 3381.01 (i..p.) | |
| 30 mg/kg | 19.4±1.37 | 30 mg/kg | 22.4±2.07 |
| 45 mg/kg | 20.2±1.73 | 37 mg/kg | 26.2±2.22 |
| 60 mg/kg | 26.9±2.45 ** | 45 mg/kg | 39.6±3.56 ** |
| 100 mg/kg | 37.7±3.17** | 60 mg/kg | 42.8±3.27** |

| | | | |
|---|---|---|---|
| ***P* <.01, vs. vehicle-treated animals | | | |

### Example 5- Evaluation of the side effect in the rotarod test

The side-effect profile of CHF 3381 was then evaluated in the rotarod test both in mice and rats. The day before the execution of the test, mice and rats were trained to maintain their equilibrium on the test apparatus. For mice, training consisted of 3 subsequent 2 min attempts on a rod rotating from 4.5 r.p.m. to 16.5 r.p.m.; for rats, of 3 subsequent 1 min attempts at 8 rpm (Kinnard and Carr, 1957). The morning of the test day, mice and rats were again tested on the rotarod and only animals able to maintain their equilibrium on the rod were retained for the experimental procedure. CHF 3381 was administered p.o. or i.p. to groups of at least 8 animals 15 min (time of peak effect for neurotoxicity) before the execution of the test. All controls received the corresponding vehicle. The number of mice falling during a 2-min test period and the number of rats falling for 3 subsequent 1-min attempts were used for the calculation of the respective doses at which 50% of the animals display neurotoxicity (TD₅₀). After oral administration CHF 3381 produced motor impairment in the rotarod test at high doses, being the TD₅₀ values calculated 233 mg/kg and 299 mg/kg in mice and rats, respectively. After i.p. administration, CHF 3381 exerted a neurotoxic effect at lower doses, being the TD₅₀ values 96 mg/kg and 113 mg/kg in mice and rats, respectively.

These results clearly show that the CHF 3381 antihyperalgesic actions appear not to be compromised by serious side-effects since CHF 3381 induces sedation and ataxia and at doses substantially higher than those endowed with an antihyperalgesic activity.

## Claims

1. Use of a compound represented by the general formula I: 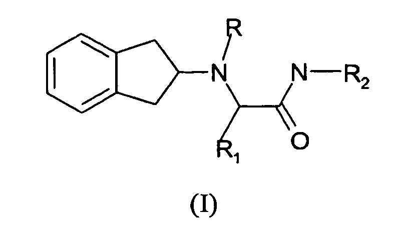 wherein:
R is hydrogen or C₁-C₄ alkyl groups;
R₁ is hydrogen, C₁-C₁₀ alkyl or optionally acylated C₁-C₄ hydroxyalkyl wherein the acylated C₁-C₄ hydroxyalkyl is selected from the group of acetyloxy C₁-C₄ alkyl, propanoyloxy C₁-C₄ alkyl, 2-methylpropanoyloxy C₁-C₄ alkyl, and benzoyloxy C₁₋C₄ alkyl;
R₂ is hydrogen; C₁-C₁₀ alkyl; phenyl; phenyl C₁-C₁₀ alkyl; and salts thereof for the preparation of a medicament for the treatment of chronic pain.

2. The use of a compound according to claim 1 wherein R is H, R₁ is H or C₁₋C₁₀ alkyl, R₂ is H or C₁-C₁₀ alkyl.

3. The use of a compound according to claim 1 or 2 wherein R, R₁ and R₂ are hydrogen.

4. The use of a compound as defined in any one of claims 1-3 wherein chronic pain is caused by a disease selected from arthritis, cancer, migraine headaches and fibromyalgia.

5. The use of a compound as defined in any one of claims 1-3 wherein chronic pain is neuropathic pain.

6. The use according to claim 5 wherein the neuropathic pain is the pain present in patients with chronic low-back pain or associated with postherpetic neuralgia, trigeminal neuralgia, diabetic neuropathy, and nerve destruction by the human immunodeficiency virus.

7. The use of the compound represented by the general formula I : 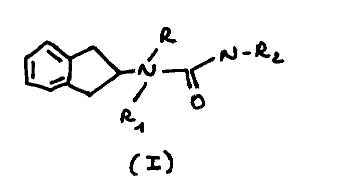 wherein R, R₁ and R₂ are hydrogen, for the preparation of pharmaceutical compositions for the treatment of acute pain.

8. The use of the compound represented by the general formula I : 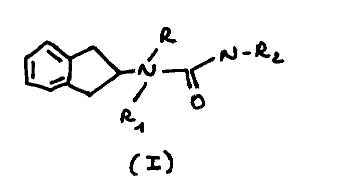 wherein R, R₁ and R₂ are hydrogen, for the preparation of pharmaceutical compositions for the treatment of inflammatory pain.

## Patentansprüche

1. Verwendung einer Verbindung, dargestellt durch die allgemeine Formel I: 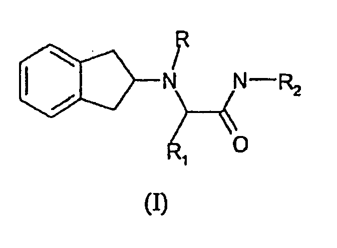 worin:
R Wasserstoff oder C₁-C₄-Alkylgruppen ist;
R₁ Wasserstoff, C₁-C₁₀-Alkyl oder gegebenenfalls acyliertes C₁₋C₄-Hydroxyalkyl ist, wobei das acylierte C₁-C₄-Hydroxyalkyl ausgewählt ist aus der Gruppe von Acetyloxy-C₁-C₄-alkyl, Propanoyloxy-C₁-C₄-alkyl, 2-Methylpropanoyloxy-C₁-C₄-alkyl und Benzoyloxy-C₁-C₄-alkyl;
R₂ Wasserstoff; C₁-C₁₀-Alkyl; Phenyl; Phenyl-C₁-C₁₀-alkyl ist; und von Salzen davon zur Herstellung eines Medikamentes für die Behandlung von chronischem Schmerz.

2. Verwendung einer Verbindung gemäß Anspruch 1, worin R H ist, R₁ H oder C₁-C₁₀-Alkyl ist, R₂ H oder C₁-C₁₀-Alkyl ist.

3. Verwendung einer Verbindung gemäß Anspruch 1 oder 2, worin R, R₁ und R₂ Wasserstoff sind.

4. Verwendung einer Verbindung, wie in einem der Ansprüche 1-3 definiert, wobei der chronische Schmerz durch eine Krankheit verursacht wird, ausgewählt aus Arthritis, Krebs, Migräne-Kopfschmerzen und Fibromyalgie.

5. Verwendung einer Verbindung, wie in einem der Ansprüche 1-3 definiert, wobei der chronische Schmerz neuropathischer Schmerz ist.

6. Verwendung gemäß Anspruch 5, wobei der neuropathische Schmerz der Schmerz ist, der bei Patienten mit chronischem Kreuz- bzw. Lendenschmerz vorhanden ist oder mit nach einem Herpes auftretender Neuralgie, Trigeminusneuralgie, diabetischer Neuropathie und Nervenzerstörung durch das Humanes Immundefizienz-Virus assoziiert ist.

7. Verwendung der Verbindung, dargestellt durch die allgemeine Formel I: 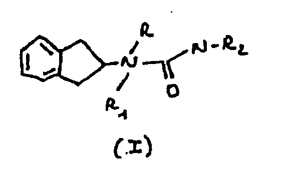 worin R, R₁ und R₂ Wasserstoff sind, zur Herstellung pharmazeutischer Zusammensetzungen für die Behandlung von akutem Schmerz.

8. Verwendung der Verbindung, dargestellt durch die allgemeine Formel I: 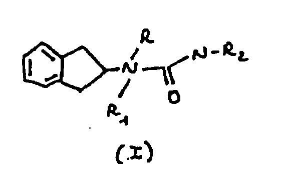 worin R, R₁ und R₂ Wasserstoff sind, zur Herstellung pharmazeutischer Zusammensetzungen für die Behandlung von Entzündungsschmerz.

## Revendications

1. Utilisation d'un composé représenté par la formule générale 1 : 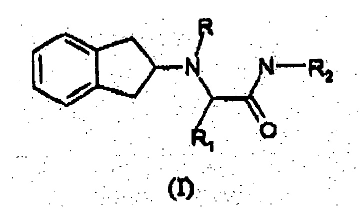 dans laquelle :
R est de l'hydrogène ou des groupes alkyles en C₁ à C₄ ;
R₁ est de l'hydrogène, alkyle en C₁ à C₁₀ ou hydroxyalkyle en C₁ à C₄ facultativement acylé, dans lequel le groupe hydroxyalkyle en C₁ à C₄ acylé est choisi dans le groupe d'acétyloxy alkyle en C₁ à C₄, propanoyloxy alkyle en C₁ à C₄, 2-méthylpropanoyloxy alkyle en C₁ à C₄ et benzoyloxy alkyle en C₁ à C₄ ;
R₂ est de l'hydrogène ; alkyle en C₁ à C₁₀ ; phényle ; phényl alkyle en C₁ à C₁₀;
et de sels de celui-ci pour la préparation d'un médicament pour le traitement d'une douleur chronique.

2. Utilisation d'un composé selon la revendication 1, dans laquelle R est H, R₁ est H ou alkyle en C₁ à C₁₀, R₂ est H ou alkyle en C₁ à C₁₀.

3. Utilisation d'un composé selon la revendication 1 ou 2, dans laquelle R, R₁ et R₂ sont de l'hydrogène.

4. Utilisation d'un composé tel que défini dans l'une quelconque des revendications 1 à 3, dans laquelle la douleur chronique est provoquée par une maladie choisie parmi l'arthrite, le cancer, les maux de tête migraineux et une fibromyalgie.

5. Utilisation d'un composé tel que défini dans l'une quelconque des revendications 1 à 3, dans laquelle la douleur chronique est une douleur neuropathique.

6. Utilisation selon la revendication 5, dans laquelle la douleur neuropathique est la douleur présente chez des patients ayant une douleur lombaire chronique ou associée avec une algie postzostérienne, une névralgie du trijumeau, une neuropathie diabétique, et une destruction nerveuse par le virus de l'immunodéficience humaine.

7. Utilisation du composé représenté par la formule générale I : 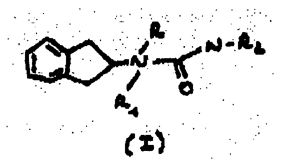 dans laquelle R, R₁ et R₂ sont de l'hydrogène, pour la préparation de compositions pharmaceutiques pour le traitement d'une douleur aiguë.

8. Utilisation du composé représenté par la formule générale 1 : 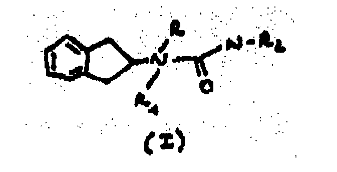 dans laquelle R, R₁ et R₂ sont de l'hydrogène, pour la préparation de compositions pharmaceutiques pour le traitement d'une douleur inflammatoire.
